# EUROPEAN PATENT SPECIFICATION

(11) **EP 2 365 072 B1**
(45) Date of publication and mention of the grant of the patent: **18.10.2017**
(21) Application number: 11160073.0
(22) Date of filing: 10.02.2005
(51) Int. Cl.: C12N 15/00, C12N 15/09, C12N 15/63

(54) **Recombinant dna for gene suppression**
Rekombinante DNA zur Genunterdrückung
ADN recombinant pour la suppression de gènes

(30) Priority: 10.02.2004 US 543157 P; 10.02.2004 US 543187 P; 11.08.2004 US 600859 P
(43) Date of publication of application: 14.09.2011
(62) Divisional of application: 05713459.5
(73) Proprietor: Monsanto Technology LLC, St. Louis, MO 63167 (US)
(72) Inventor: Malvar, Thomas, M., North Stonington, CT 06359 (US); Huang, Shihshieh, St. Louis, Missouri 63167 (US); Luethy, Michael, H., St. Louis, Missouri 63167 (US)
(74) Representative: dompatent von Kreisler Selting Werner - Partnerschaft von Patent- und Rechtsanwälten mbB

(56) References cited:
- EP-A- 0 428 881
- WO-A-03/077643
- WO-A-03/078629
- WO-A-2005/077117
- WO-A-2007/024207
- WO-A1-99/53050
- WO-A1-99/61631
- US-A1- 2003 036 197
- HUANG SHIHSHIEH ET AL: "High lysine and high tryptophan transgenic maize resulting from the reduction of both 19- and 22-kD alpha-zeins", PLANT MOLECULAR BIOLOGY, vol. 61, no. 3, June 2006 (2006-06), pages 525-535, XP002485016, ISSN: 0167-4412
- ANONYMOUS: "About CSIRO' s HairpinRNAi", INTERNET ARTICLE, [Online] XP002485017, Retrieved from the Internet: URL:http://www.pi.csiro.au/rnai/about.htm>
- REBOWSKI GRZEGORZ ET AL: "Antisense hairpin loop oligonucleotides as inhibitors of expression of multidrug resistance-associated protein 1: Their stability in fetal calf serum and human plasma", ACTA BIOCHIMICA POLONICA, vol. 48, no. 4, 2001, pages 1061-1076, XP002485018, ISSN: 0001-527X
- SMITH NA ET AL: "Total silencing by intron-spliced hairpin RNAs", NATURE, NATURE PUBLISHING GROUP, LONDON, GB, vol. 407, no. 6802, 21 September 2000 (2000-09-21), pages 319-320, XP002187667, ISSN: 0028-0836, DOI: DOI:10.1038/35036500
- WATERHOUSE ET AL: "Virus resistance and gene silencing in plants can be induced by simultaneous expression of sense and antisense RNA", PROCEEDINGS OF THE NATIONAL ACADEMY OF SCIENCES, NATIONAL ACADEMY OF SCIENCES, WASHINGTON, DC; US, vol. 95, 1 November 1998 (1998-11-01), pages 13959-13964, XP002114472, ISSN: 0027-8424, DOI: DOI:10.1073/PNAS.95.23.13959
- WESLEY S VARSHA ET AL: "Construct design for efficient, effective and high-throughput gene silencing in plants", PLANT JOURNAL, BLACKWELL SCIENTIFIC PUBLICATIONS, OXFORD, GB, vol. 27, no. 6, 1 September 2001 (2001-09-01), pages 581-590, XP002187670, ISSN: 0960-7412, DOI: DOI:10.1046/J.1365-313X.2001.01105.X

### SEQUENCE LISTING

<110> Monsanto Technology, LLC
<120> Recombinant DNA for Gene Suppression
<130> 38-15 (53428)B
<150> EP 05713459.5
   <151> 2005-02-10
<150> US 60/543,157
   <151> 2004-02-10
<150> US 60/543,187
   <151> 2004-02-10
<150> US 60/600,859
   <151> 2004-08-11
<160> 2
<170> PatentIn version 3.2
<210> 1
   <211> 8590
   <212> DNA
   <213> Artificial
<220>
   <223> recombinant DNA construct in plasmid between Agrabacterium borders
<400> 1
   aggatttttc ggcgctgcgc tacgtccgcg accgcgttga gggatcaagc cacagcagcc      60
   cactcgacct tctagccgac ccagacgagc caagggatct ttttggaatg ctgctccgtc     120
   gtcaggcttt ccgacgtttg ggtggttgaa cagaagtcat tatcgcacgg aatgccaagc    180
   actcccgagg ggaaccctgt ggttggcatg cacatacaaa tggacgaacg gataaacctt    240
   ttcacgccct tttaaatatc cgattattct aataaacgct cttttctctt aggtttaccc    300
   gccaatatat cctgtcaaac actgatagtt taaactgaag gcgggaaacg acaatctgat    360
   ccccatcaag cttactcgag gtcattcata tgcttgagaa gagagtcggg atagtccaaa    420
   ataaaacaaa ggtaagatta cctggtcaaa agtgaaaaca tcagttaaaa ggtggtataa    480
   agtaaaatat cggtaataaa aggtggccca aagtgaaatt tactcttttc tactattata    540
   aaaattgagg atgtttttgt cggtactttg atacgtcatt tttgtatgaa ttggttttta    600
   agtttattcg cttttggaaa tgcatatctg tatttgagtc gggttttaag ttcgtttgct    660
   tttgtaaata cagagggatt tgtataagaa atatctttag aaaaacccat atgctaattt     720
   gacataattt ttgagaaaaa tatatattca ggcgaattct cacaatgaac aataataaga 780
   ttaaaatagc tttcccccgt tgcagcgcat gggtattttt tctagtaaaa ataaaagata 840
   aacttagact caaaacattt acaaaaacaa cccctaaagt tcctaaagcc caaagtgcta 900
   tccacgatcc atagcaagcc cagcccaacc caacccaacc cagcccaccc cagtccagcc 960
   aactggacaa tagtctccac acccccccac tatcaccgtg agttgtccgc acgcaccgca 1020
   cgtctcgcag ccaaaaaaaa aaagaaagaa aaaaaagaaa aagaaaaaac agcaggtggg 1080
   tccgggtcgt gggggccgga aacgcgagga ggatcgcgag ccagcgacga ggccggccct 1140
   ccctccgctt ccaaagaaac gccccccatc gccactatat acataccccc ccctctcctc 1200
   ccatcccccc aaccctacca ccaccaccac caccacctcc acctcctccc ccctcgctgc 1260
   cggacgacga gctcctcccc cctccccctc cgccgccgcc gcgccggtaa ccaccccgcc 1320
   cctctcctct ttctttctcc gttttttttt ccgtctcggt ctcgatcttt ggccttggta 1380
   gtttgggtgg gcgagaggcg gcttcgtgcg cgcccagatc ggtgcgcggg aggggcggga 1440
   tctcgcggct ggggctctcg ccggcgtgga tccggcccgg atctcgcggg gaatggggct 1500
   ctcggatgta gatctgcgat ccgccgttgt tgggggagat gatggggggt ttaaaatttc 1560
   cgccgtgcta aacaagatca ggaagagggg aaaagggcac tatggtttat atttttatat 1620
   atttctgctg cttcgtcagg cttagatgtg ctagatcttt ctttcttctt tttgtgggta 1680
   gaatttgaat ccctcagcat tgttcatcgg tagtttttct tttcatgatt tgtgacaaat 1740
   gcagcctcgt gcggagcttt tttgtaggta gaagtgatca accatggcgc aagttagcag 1800
   aatctgcaat ggtgtgcaga acccatctct tatctccaat ctctcgaaat ccagtcaacg 1860
   caaatctccc ttatcggttt ctctgaagac gcagcagcat ccacgagctt atccgatttc 1920
   gtcgtcgtgg ggattgaaga agagtgggat gacgttaatt ggctctgagc ttcgtcctct 1980
   taaggtcatg tcttctgttt ccacggcgtg catgcttcac ggtgcaagca gccggcccgc 2040
   aaccgcccgc aaatcctctg gcctttccgg aaccgtccgc attcccggcg acaagtcgat 2100
   ctcccaccgg tccttcatgt tcggcggtct cgcgagcggt gaaacgcgca tcaccggcct 2160
   tctggaaggc gaggacgtca tcaatacggg caaggccatg caggcgatgg gcgcccgcat 2220
   ccgtaaggaa ggcgacacct ggatcatcga tggcgtcggc aatggcggcc tcctggcgcc 2280
   tgaggcgccg ctcgatttcg gcaatgccgc cacgggctgc cgcctgacga tgggcctcgt 2340
   cggggtctac gatttcgaca gcaccttcat cggcgacgcc tcgctcacaa agcgcccgat 2400
   gggccgcgtg ttgaacccgc tgcgcgaaat gggcgtgcag gtgaaatcgg aagacggtga 2460
   ccgtcttccc gttaccttgc gcgggccgaa gacgccgacg ccgatcacct accgcgtgcc 2520
   gatggcctcc gcacaggtga agtccgccgt gctgctcgcc ggcctcaaca cgcccggcat 2580
   cacgacggtc atcgagccga tcatgacgcg cgatcatacg gaaaagatgc tgcagggctt 2640
   tggcgccaac cttaccgtcg agacggatgc ggacggcgtg cgcaccatcc gcctggaagg 2700
   ccgcggcaag ctcaccggcc aagtcatcga cgtgccgggc gacccgtcct cgacggcctt 2760
   cccgctggtt gcggccctgc ttgttccggg ctccgacgtc accatcctca acgtgctgat 2820
   gaaccccacc cgcaccggcc tcatcctgac gctgcaggaa atgggcgccg acatcgaagt 2880
   catcaacccg cgccttgccg gcggcgaaga cgtggcggac ctgcgcgttc gctcctccac 2940
   gctgaagggc gtcacggtgc cggaagaccg cgcgccttcg atgatcgacg aatatccgat 3000
   tctcgctgtc gccgccgcct tcgcggaagg ggcgaccgtg atgaacggtc tggaagaact 3060
   ccgcgtcaag gaaagcgacc gcctctcggc cgtcgccaat ggcctcaagc tcaatggcgt 3120
   ggattgcgat gagggcgaga cgtcgctcgt cgtgcgtggc cgccctgacg gcaaggggct 3180
   cggcaacgcc tcgggcgccg ccgtcgccac ccatctcgat caccgcatcg ccatgagctt 3240
   cctcgtcatg ggcctcgtgt cggaaaaccc tgtcacggtg gacgatgcca cgatgatcgc 3300
   cacgagcttc ccggagttca tggacctgat ggccgggctg ggcgcgaaga tcgaactctc 3360
   cgatacgaag gctgcctgat gagctcgaat tcccgatcgt tcaaacattt ggcaataaag 3420
   tttcttaaga ttgaatcctg ttgccggtct tgcgatgatt atcatataat ttctgttgaa 3480
   ttacgttaag catgtaataa ttaacatgta atgcatgacg ttatttatga gatgggtttt 3540
   tatgattaga gtcccgcaat tatacattta atacgcgata gaaaacaaaa tatagcgcgc 3600
   aaactaggat aaattatcgc gcgcggtgtc atctatgtta ctagatcggg gatgggggat 3660
   ccactagtga tatccgtcga gtggcggccg cgttttatga ataataataa tgcatatctg 3720
   tgcattacta cctgggatac aagggcttct ccgccataac aaattgagtt gcgatgctga 3780
   gaacgaacgg ggaagaaagt aagcgccgcc caaaaaaaac gaacatgtac gtcggctata 3840
   gcaggtgaaa gttcgtgcgc caatgaaaag ggaacgatat gcgttgggta gttgggatac 3900
   ttaaatttgg agagtttgtt gcatacacta atccactaaa gttgtctatc tttttaacag 3960
   ctctaggcag gatataagat ttatatctaa tctgttggag ttgcttttag agtaactttt 4020
   ctctctgttt cgtttatagc cgattagcac aaaattaaac taggtgacga gaaataaaga 4080
   aaaacggagg cagtaaaaaa tacccaaaaa aatacttgga gatttttgtc tcaaaattat 4140
   cttctaattt taaaagctac atattaaaaa tactatatat taaaaatact tcgagatcat 4200
   tgcttgggat gggcagggcc aatagctaat tgctaaggat gggctatatt tatgtatcgt 4260
   ctgaaacatg taggggctaa tagttagatg actaatttgc tgtgttcgta cggggtgctg 4320
   tttgagccta gcgatgaagg gtcatagttt catacaagaa ctcacttttg gttcgtctgc 4380
   tgtgtctgtt ctcagcgtaa cggcatcaat ggatgccaaa ctccgcaagg ggacaaatga 4440
   agaagcgaag agattataga acacgcacgt gtcattattt atttatggac ttgcctcagt 4500
   agcttacagc atcgtacccg cacgtacata ctacagagcc acacttattg cactgcctgc 4560
   cgcttacgta catagttaac acgcagagag gtatatacat acacgtccaa cgtctccact 4620
   caggctcatg ctacgtacgc acgtcggtcg cgcgccaccc tctcgttgct tcctgctcgt 4680
   tttggcgaat tccgatttgg caagtgttcc agagcaaaag ctggaagctc tcgtagtctg 4740
   agcctctttg ctgattcata caagttatga ccatctacat ggatcgtctc accaagaaat 4800
   ttgtagactg caggattttt ccctgaccgg agtgcaccag ctgggttcca actgaattta 4860
   taggcaagcg gattgtttgc tgcagctgga gatggcaatc caccacagta agatgtaaat 4920
   gcctttattt ttccctttcg tgcatgagct tcatcaatca tcttcattga catcaagtga 4980
   tctatgccag gatctaggcc catttcacaa agtatagtta cacctgcatc tttggcagct 5040
   tggctcaagt ttgacatgga ttcatcaaca tagcttgccg ttaccatgtg cttcttcaac 5100
   tctatgcata ctcctgcaat ggcagcatga aaactagcag gcagcaccgg ttggacatca 5160
   ttgagacagc tggaggttca tttcacttgg ttagatgtga agttggacaa agcacggatg 5220
   atatgtcgta ctcagagctt gaagtaggag cagatgatac tgccacattg gataaaatta 5280
   ttgattcctt gacttcttta gctaatgaac atggtggaga tcacgatgcc gggcaagaaa 5340
   ttgaattagc tctgaagata ggaaaagtca atgagtatga aactgacgtc acaattgata 5400
   aaggagggcc aaagatttta attcttggag ctggaagagt ctgtcggcca gctgctgagt 5460
   ttctggcatc ttacccagac atatgtacct atggtgttga tgaccatgat gcagatcaaa 5520
   ttcatgttat cgtggcatct ttgtatcaaa aagatgcaga agagacagtt gatggtattg 5580
   aaaatacaac tgctacccag cttgatgttg ctgatattgg aagcctttca gatcttgttt 5640
   ctcaggttga ggttgtaatt agcttgctgc ctgctagttt tcatgctgcc attgcaggag 5700
   tatgcataga gttgaagaag cacatggtaa cggcaagcta tgttgatgaa tccatgtcaa 5760
   acttgagcca agctgccaaa gatgcaggtg taactatact ttgtgaaatg ggcctagatc 5820
   ctggcataga tcacttgatg tcaatgaaga tgattgatga agctcatgca cgaaagggaa   5880
   aaataaaggc atttacatct tactgtggtg gattgccatc tccagctgca gcaaacaatc   5940
   cgcttgccta taaattcagt tggaacccag ctggtgcact ccggtcaggg aaaaatcctg   6000
   cagtctacaa atttcttggt gagacgatcc atgtagatgg tcataacttg tatgaatcag   6060
   caaagaggct cagactacga gagcttccag cttttgctct ggaacacttg ccaaatcggg   6120
   atccgcagct gcacgggtcc aggaaagcaa tcgcatagtc aagctaaatc atcaagatgc   6180
   aaacttttcg cccttgctaa acacggtaaa attcgaatgg acatgtgtgg agcagcaaag   6240
   gccttacgtc cgagaaacag ggccactcaa cgagttagtt aaattcaaag aaagaaacgc   6300
   ctccttgcaa gttgcaacat tcttagatca tactgatgaa aatgacgtct ttcattaaag   6360
   aacagggaag atagatcttt gctcaatatc gtatgatgtg ttcagccaga ctgtcggatg   6420
   gaccacacgg taatagcagt gctggacgat gttacatcga gaaagattac tagccttttc   6480
   atgggagtga aggatataaa agaaataagt tcaccacgat tgcaggatag catacaagat   6540
   cagcgccact gcggcactgt tcatcgaaaa aaaaactgtg gacgaagcta gctttcccca   6600
   aaattactca acgaatcata aaccaagatt agtcagatca agagacagag gagaaacaag   6660
   gcggaccttt gcacttgatc ggatccttgg gttggctgta tgcagaacta aagcggaggt   6720
   ggcgcgcatt tataccagcg ccgggccctg gtacgtggcg cggccgcgcg gctacgtgga   6780
   ggaaggctgc gtggcagcag acacacgggt cgccacgtcc cgccgtactc tccttaccgt   6840
   gcttatccgg gctccggctc ggtgcacgcc agggtgtggc cgcctctgag cagactttgt   6900
   cgtgttccac agtggtgtcg tgttccgggg actccgatcc gcggcgagcg accgagcgtg   6960
   taaaagagtt cctactaggt acgttcattg tatctggacg acgggcagcg gacaatttgc   7020
   tgtaagagag gggcagtttt tttttagaaa aacagagaat tccgttgagc taattgtaat   7080
   tcaacaaata agctattagt tggttttagc ttagattaaa gaagctaacg actaatagct   7140
   aataattagt tggtctatta gttgactcat tttaaggccc tgtttcaatc tcgcgagata   7200
   aactttagca gctatttttt agctactttt agccatttgt aatctaaaca ggagagctaa   7260
   tggtggtaat tgaaactaaa ctttagcact tcaattcata tagctaaagt ttagcaggaa   7320
   gctaaacttt atcccgtgag attgaaacgg ggcctaaatc tctcagctat ttttgatgca   7380
   aattactgtc actactggaa tcgagcgctt tgccgagtgt caaagcctga aaaacactcc   7440
   gtaaagactt tgcctagtgt gacactcgac aaagagatct cgacgaacag tacatcgaca   7500
   acggcttctt tgtcgagtac tttttatcgg acacttgaca aagtctttgt cgagtgaact   7560
   acattgaaac tctatgattt tatgtgtagg tcacttaggt ttctacacat agtacgtcac   7620
   aactttaccg aaacattatc aaatttttat cacaacctct atatatgata tcatgacatg   7680
   tggacaagtt tcattaattt ctgactttat ttgtgtttta tacaattttt aaacaactag   7740
   ataacaagtt cacggtcatg tttagtgagc atggtgcttg aagattctgg tctgcttctg   7800
   aaatcggtcg taacttgtgc tagataacat gcatatcatt tattttgcat gcacggtttt   7860
   ccatgtttcg agtgacttgc agtttaaatg tgaattttcc gaagaaattc aaataaacga   7920
   actaaatcta atatttatag aaaacatttt tgtaaatatg taattgtgcc aaaatggtac   7980
   atgtagatct acatagtgta ggaacatacc acaaaaagtt tggttggcaa aataaaaaaa   8040
   ataaaatata ctttatccga gtgtccaagg tatggcactc ggcccgggtg gccaagctta   8100
   ctagcccggg cgcgccttaa ttaagcggcc gcatcgatcg tgaagtttct catctaagcc   8160
   cccatttgga cgtgaatgta gacacgtcga aataaagatt tccgaattag aataatttgt   8220
   ttattgcttt cgcctataaa tacgacggat cgtaatttgt cgttttatca aaatgtactt   8280
   tcattttata ataacgctgc ggacatctac atttttgaat tgaaaaaaaa ttggtaatta   8340
   ctctttcttt ttctccatat tgaccatcat actcattgct gatccatgta gatttcccgg   8400
   acatgaagcc atttacaatt gaatatatcc tgccgccgct gccgctttgc acccggtgga   8460
   gcttgcatgt tggtttctac gcagaactga gccggttagg cagataattt ccattgagaa   8520
   ctgagccatg tgcaccttcc ccccaacacg gtgagcgacg gggcaacgga gtgatccaca   8580
   tgggactttt                                                          8590
<210> 2
   <211> 7794
   <212> DNA
   <213> Artificial
<220>
   <223> recombinant DNA construct in plasmid between Agrobacterium borders
<400> 2
   aggatttttc ggcgctgcgc tacgtccgcg accgcgttga gggatcaagc cacagcagcc     60
   cactcgacct tctagccgac ccagacgagc caagggatct ttttggaatg ctgctccgtc     120
   gtcaggcttt ccgacgtttg ggtggttgaa cagaagtcat tatcgcacgg aatgccaagc    180
   actcccgagg ggaaccctgt ggttggcatg cacatacaaa tggacgaacg gataaacctt    240
   ttcacgccct tttaaatatc cgattattct aataaacgct cttttctctt aggtttaccc    300
   gccaatatat cctgtcaaac actgatagtt taaactgaag gcgggaaacg acaatctgat     360
   ccccatcaag cttactcgag gtcattcata tgcttgagaa gagagtcggg atagtccaaa     420
   ataaaacaaa ggtaagatta cctggtcaaa agtgaaaaca tcagttaaaa ggtggtataa     480
   agtaaaatat cggtaataaa aggtggccca aagtgaaatt tactcttttc tactattata     540
   aaaattgagg atgtttttgt cggtactttg atacgtcatt tttgtatgaa ttggttttta     600
   agtttattcg cttttggaaa tgcatatctg tatttgagtc gggttttaag ttcgtttgct     660
   tttgtaaata cagagggatt tgtataagaa atatctttag aaaaacccat atgctaattt     720
   gacataattt ttgagaaaaa tatatattca ggcgaattct cacaatgaac aataataaga     780
   ttaaaatagc tttcccccgt tgcagcgcat gggtattttt tctagtaaaa ataaaagata     840
   aacttagact caaaacattt acaaaaacaa cccctaaagt tcctaaagcc caaagtgcta     900
   tccacgatcc atagcaagcc cagcccaacc caacccaacc caacccaccc cagtccagcc     960
   aactggacaa tagtctccac acccccccac tatcaccgtg agttgtccgc acgcaccgca    1020
   cgtctcgcag ccaaaaaaaa aaagaaagaa aaaaaagaaa aagaaaaaac agcaggtggg    1080
   tccgggtcgt gggggccgga aacgcgagga ggatcgcgag ccagcgacga ggccggccct    1140
   ccctccgctt ccaaagaaac gccccccatc gccactatat acataccccc ccctctcctc    1200
   ccatcccccc aaccctacca ccaccaccac caccacctcc acctcctccc ccctcgctgc    1260
   cggacgacga gctcctcccc cctccccctc cgccgccgcc gcgccggtaa ccaccccgcc    1320
   cctctcctct ttctttctcc gttttttttt ccgtctcggt ctcgatcttt ggccttggta    1380
   gtttgggtgg gcgagaggcg gcttcgtgcg cgcccagatc ggtgcgcggg aggggcggga    1440
   tctcgcggct ggggctctcg ccggcgtgga tccggcccgg atctcgcggg gaatggggct    1500
   ctcggatgta gatctgcgat ccgccgttgt tgggggagat gatggggggt ttaaaatttc    1560
   cgccgtgcta aacaagatca ggaagagggg aaaagggcac tatggtttat atttttatat    1620
   atttctgctg cttcgtcagg cttagatgtg ctagatcttt ctttcttctt tttgtgggta    1680
   gaatttgaat ccctcagcat tgttcatcgg tagtttttct tttcatgatt tgtgacaaat    1740
   gcagcctcgt gcggagcttt tttgtaggta gaagtgatca accatggcgc aagttagcag    1800
   aatctgcaat ggtgtgcaga acccatctct tatctccaat ctctcgaaat ccagtcaacg    1860
   caaatctccc ttatcggttt ctctgaagac gcagcagcat ccacgagctt atccgatttc    1920
   gtcgtcgtgg ggattgaaga agagtgggat gacgttaatt ggctctgagc ttcgtcctct    1980
   taaggtcatg tcttctgttt ccacggcgtg catgcttcac ggtgcaagca gccggcccgc   2040
   aaccgcccgc aaatcctctg gcctttccgg aaccgtccgc attcccggcg acaagtcgat   2100
   ctcccaccgg tccttcatgt tcggcggtct cgcgagcggt gaaacgcgca tcaccggcct   2160
   tctggaaggc gaggacgtca tcaatacggg caaggccatg caggcgatgg gcgcccgcat   2220
   ccgtaaggaa ggcgacacct ggatcatcga tggcgtcggc aatggcggcc tcctggcgcc   2280
   tgaggcgccg ctcgatttcg gcaatgccgc cacgggctgc cgcctgacga tgggcctcgt   2340
   cggggtctac gatttcgaca gcaccttcat cggcgacgcc tcgctcacaa agcgcccgat   2400
   gggccgcgtg ttgaacccgc tgcgcgaaat gggcgtgcag gtgaaatcgg aagacggtga   2460
   ccgtcttccc gttaccttgc gcgggccgaa gacgccgacg ccgatcacct accgcgtgcc   2520
   gatggcctcc gcacaggtga agtccgccgt gctgctcgcc ggcctcaaca cgcccggcat   2580
   cacgacggtc atcgagccga tcatgacgcg cgatcatacg gaaaagatgc tgcagggctt   2640
   tggcgccaac cttaccgtcg agacggatgc ggacggcgtg cgcaccatcc gcctggaagg   2700
   ccgcggcaag ctcaccggcc aagtcatcga cgtgccgggc gacccgtcct cgacggcctt   2760
   cccgctggtt gcggccctgc ttgttccggg ctccgacgtc accatcctca acgtgctgat   2820
   gaaccccacc cgcaccggcc tcatcctgac gctgcaggaa atgggcgccg acatcgaagt   2880
   catcaacccg cgccttgccg gcggcgaaga cgtggcggac ctgcgcgttc gctcctccac   2940
   gctgaagggc gtcacggtgc cggaagaccg cgcgccttcg atgatcgacg aatatccgat   3000
   tctcgctgtc gccgccgcct tcgcggaagg ggcgaccgtg atgaacggtc tggaagaact   3060
   ccgcgtcaag gaaagcgacc gcctctcggc cgtcgccaat ggcctcaagc tcaatggcgt   3120
   ggattgcgat gagggcgaga cgtcgctcgt cgtgcgtggc cgccctgacg gcaaggggct   3180
   cggcaacgcc tcgggcgccg ccgtcgccac ccatctcgat caccgcatcg ccatgagctt   3240
   cctcgtcatg ggcctcgtgt cggaaaaccc tgtcacggtg gacgatgcca cgatgatcgc   3300
   cacgagcttc ccggagttca tggacctgat ggccgggctg ggcgcgaaga tcgaactctc   3360
   cgatacgaag gctgcctgat gagctcgaat tcccgatcgt tcaaacattt ggcaataaag   3420
   tttcttaaga ttgaatcctg ttgccggtct tgcgatgatt atcatataat ttctgttgaa   3480
   ttacgttaag catgtaataa ttaacatgta atgcatgacg ttatttatga gatgggtttt   3540
   tatgattaga gtcccgcaat tatacattta atacgcgata gaaaacaaaa tatagcgcgc   3600
   aaactaggat aaattatcgc gcgcggtgtc atctatgtta ctagatcggg gatgggggat   3660
   ccactagtga tatccgtcga ctggtaccta cgcgtagcta gcccgggcgc gccttaatta   3720
   agcggccgct tcgagtggct gcaggtcgat tgatgcatgt tgtcaatcaa ttggcaagtc    3780
   ataaaatgca ttaaaaaata ttttcatact caactacaaa tccatgagta taactataat    3840
   tataaagcaa tgattagaat ctgacaagga ttctggaaaa ttacataaag gaaagttcat    3900
   aaatgtctaa aacacaagag gacatacttg tattcagtaa catttgcagc ttttctaggt    3960
   ctgaaaatat atttgttgcc tagtgaataa gcataatggt acaactacaa gtgttttact    4020
   cctcatatta acttcggtca ttagaggcca cgatttgaca catttttact caaaacaaaa    4080
   tgtttgcata tctcttataa tttcaaattc aacacacaac aaataagaga aaaaacaaat    4140
   aatattaatt tgagaatgaa caaaaggacc atatcattca ttaactcttc tccatccatt    4200
   tccatttcac agttcgatag cgaaaaccga ataaaaaaca cagtaaatta caagcacaac    4260
   aaatggtaca agaaaaacag ttttcccaat gccataatac tcaaactcag taggattctg    4320
   gtgtgtgcgc aatgaaactg atgcattgaa cttgacgaac gttgtcgaaa ccgatgatac    4380
   gaacgaaagc tgaattccta gctggctgaa tggtagtagt tgttgctgct gtaaataagc    4440
   aggagagttc aatgctgtca gttggttgaa tggaagaaat tgctgggggt aggcagcaga    4500
   tagctggctg aatggtagtt gttgttgttg caaataagaa gcagagttca atgcagctag    4560
   ttggttgaat ggaagaaact gctgttgctg agagtaggca gcaaggtttg ctagcacaag    4620
   ttgttgtagt tgttgtgccc tgatgttttg tgccaataaa tgcaccaaag gtaactgctg    4680
   taatagggct gatgattgtt ggaggaacaa gggtgataaa ggtaagatgc cagctgcgat    4740
   tgcctgttat gcataaagat ggcacctcca acgatgggtt gctgcaaggc agggttcatc    4800
   aaagagaact ggttgtatgg cagcaattgt tgttgctgct gcaggaaggt agcgaccaat    4860
   gggttagcca ctgccaatgg attaagtaac tgttgtcgct gttgtaggta cgcagcagag    4920
   tttgacacag ccagttggtt gaatggaagc aactgttgta agtaggcagc agggtttgcc    4980
   acagctagct gagtcagagc tggtacaatt tgttgcagca actgttgttg taggtacgta    5040
   ggtgggcccg ctaccaagat attagccctc cttgcgcttc ttgccctttt agtgagcgca    5100
   acaaatgcgt tcattattcc acagtgctca cttgctccta gtgccagtat tccacagttc    5160
   ctcccaccag ttacttcaat gggcttcgaa catccagccg tgcaagccta caggctacaa    5220
   ctagcgcttg cggcgagcgc cttacaacaa ccaattgccc aattgcaaca acaatccttg    5280
   gcacatctaa ccctacaaac cattgcaacg caacaacaac aacaacagtt tctgccatca    5340
   ctgagccacc tagccgtggt gaaccctgtc acctacttgc aacagcagct gcttgcatcc    5400
   aacccacttg ctctggcgaa cgtagctgca taccagcaac aacaacagct gcaacagttt 5460
   atgccagtgc tcagtcaact agccatggtg aaccctgccg tctacctaca actactttca 5520
   tctagcccgc tcgcggtggg caatgcacct acgtacctac aacaacagtt gctgcaacaa 5580
   attgtaccag ctctgactca gctagctgtg gcaaaccctg ctgcctactt acaacagttg 5640
   cttccattca accaactggc tgtgtcaaac tctgctgcgt acctacaaca gcgacaacag 5700
   ttacttaatc cattggcagt ggctaaccca ttggtcgcta ccttcctgca gcagcaacaa 5760
   caattgctgc catacaacca gttctctttg atgaaccctg ccttgcagca acccatcgtt 5820
   ggaggtgcca tctttaccgg taacaggcaa tcgcagctgg catcttacct ttatcaccct 5880
   tgttcctcca acaatcatca gccctattac agcagttacc tttggtgcat ttattggcac 5940
   aaaacatcag ggcacaacaa ctacaacaac ttgtgctagc aaaccttgct gcctactctc 6000
   agcaacaaca gtttcttcca ttcaaccaac tagctgcatt gaactctgct tcttatttgc 6060
   aacaacaaca actaccattc agccagctat ctgctgccta cccccagcaa tttcttccat 6120
   tcaaccaact gacagctttg aactctcctg cttatttaca gcagcaacaa ctactaccat 6180
   tcagccagct agggatccgg taccgggttc ttctgcgctc tggagtagat aaagctaatg 6240
   gtctgaagac ccagtggtgg tgatggagaa gtgcacaggc atgcgagcgt tatttatagc 6300
   tttgattaat taacacaatt tcttgtgttc ttatgccacc gagacggctg taggcagctt 6360
   catggtttct tgccaaatgt atatgactcg tcactctctt tacgtagcac gtcgatggtt 6420
   catctggaat cattctgtac ttctgcgtgg ctcagttttg ttgccttcta caggttgttg 6480
   atctacgtaa aacgaattag atttagcttg acatatggct ttttttttgt tgtaaattta 6540
   ctttacacgt caaggatttt tgtcctgtcc ggcctatttt atttttcatg aaacgatctt 6600
   tgtaatgcaa tatgagttgt ttgtaatgtc ttgtgagctg taagcatgta tatcagatga 6660
   gtatgatctc ggcatgactc accgtgtttc tttgcacaca gagaggattt gtttgattgt   6720
   ttcttaccca atacccttga cgtgcaattt tggttgatgt tctgtgagtt gttaaggata   6780
   caacaaattc ttggagcttt acatgccaat gcatggttgt ttcgtgttcc tcaccacttt 6840
   aggacttata cggttgcacc tggatgatcg aaggggattg ggagagatta aatctccttc 6900
   tattcaattt tgactaggaa gagatttaat cgtttccaac ccctttcgat ccagacgtaa 6960
   gcgaacaagt tttttatttg gataccctct tattcatctt aatacacaca tgtattaagt 7020
   tgcactagtt atatgcccgt gcattgctac ggtttatata tatatatata tatatgtata 7080
   tatatatata tgatatatga taaattttgt tttaataaaa catatgtttt ctattgatta 7140
   ggttgtgtga atatggagcc aacaaccaat atccagaaca cttatacata atttcacctt   7200
   attttgtaca taaactctct tattatagta gtagagaaga gattataaga gtgcgggttg   7260
   attataaaga aatgtaggag ttttttaata atattgacgc gggacaagct tactagtagc   7320
   ttgttaacgc ggccgcatcg atcgtgaagt ttctcatcta agcccccatt tggacgtgaa   7380
   tgtagacacg tcgaaataaa gatttccgaa ttagaataat ttgtttattg ctttcgccta   7440
   taaatacgac ggatcgtaat ttgtcgtttt atcaaaatgt actttcattt tataataacg   7500
   ctgcggacat ctacattttt gaattgaaaa aaaattggta attactcttt ctttttctcc   7560
   atattgacca tcatactcat tgctgatcca tgtagatttc ccggacatga agccatttac   7620
   aattgaatat atcctgccgc cgctgccgct ttgcacccgg tggagcttgc atgttggttt   7680
   ctacgcagaa ctgagccggt taggcagata atttccattg agaactgagc catgtgcacc   7740
   ttccccccaa cacggtgagc gacggggcaa cggagtgatc cacatgggac tttt         7794

## Description

### Field of the Invention

Disclosed herein are recombinant DNA constructs for producing gene-suppressing loops of anti-sense RNA and methods of using such constructs and transgenic plants expressing gene-suppressing loops of anti-sense RNA.

### Background

Certain plants have low levels of specific amino acids compared to other plants, e.g. corn has low levels of lysine, methionine and tryptophan. Efforts to increase amino acid levels in transgenic plants include expressing recombinant DNA which encodes proteins in an amino acid synthesis pathway at higher levels than native genes. One such gene for producing enhanced levels of lysine in corn is a bacterial dihydropicolinic acid synthase. A concept for even more enhanced levels of amino acids includes suppression of genes encoding proteins in amino acid catabolic pathways.

Gene suppression includes any of the well-known methods for suppressing transcription of a gene or the accumulation of the mRNA corresponding to that gene thereby preventing translation of the transcript into protein. More particularly, gene suppression mediated by inserting a recombinant DNA construct with anti-sense oriented DNA to regulate gene expression in plant cells is disclosed in U.S. Patent 5,107,065 (Shewmaker et al.) and US Patent 5,759,829 (Shewmaker et al.). Plants transformed using such anti-sense oriented DNA constructs for gene suppression can comprise integrated DNA arranged as an inverted repeat that resulted from co-insertion of several copies of the transfer DNA (T-DNA) into plants by *Agrobacterium-mediated* transformation, as disclosed by Redenbaugh et al_ in "Safety Assessment of Genetically Engineered Flavr SavrTM Tomato, CRC Press, Inc. (1992). Inverted repeat insertions can comprise a part or all of the T-DNA, e.g. contain an inverted repeat of a complete or partial anti-sense construct. Screening for inserted DNA comprising inverted repeat elements can improve the efficiency of identifying transformation events effective for gene silencing when the transformation construct is a simple anti-sense DNA construct.

Gene suppression triggered by inserting a recombinant DNA construct with sense-oriented DNA to regulate gene expression in plants is disclosed in U.S. Patent 5,283,184 (Jorgensen et al.) and U.S. Patent 5,231,020 (Jorgensen et al.). Inverted T-DNA providing gene suppression in plants transformed with such sense constructs by *Agrobacterium* is organized predominately in inverted repeat structures, as disclosed by Jorgensen et al., Mol. Gen. Genet., 207: 471-477 (1987). See also Stam et al., The Plant Journal, 12: 63-82 (1997) and De Buck et al., Plant Mol. Biol. 46 433-445 (2001), who used segregation studies to support Jorgensen's finding that in many events gene silencing is mediated by multimeric transgene T-DNA where the T-DNAs are arranged in inverted repeats_Screening for inserted DNA comprising inverted repeat elements can improve the gene silencing efficiency when transforming with simple sense-orientated DNA constructs.

Gene silencing can also be effected by transcribing RNA from both a sense and an anti-sense oriented DNA using two separate transcription units, e.g. as disclosed by Shewmaker et al. in U.S. Patent 5,107,065 where in Example 1 a binary vector was prepared with both sense and anti-sense *aroA* genes. Similar constructs are disclosed in International Publication No. WO 99/53050 (Waterhouse et al.*)*. See also U.S. Patent 6,326,193 where gene targeted DNA is operably linked to opposing promoters. Gene suppression can be achieved in plants by providing transformation constructs that are capable of generating an RNA that can form double-stranded RNA along at least part of its length. Gene suppression in plants is disclosed in EP 0426195 A1 (Goldbach et al.*)* where recombinant DNA constructs for transcription into hairpin RNA provided transgenic plants with resistance to tobacco spotted wilt virus. WO 03/078629 and WO 03/077643 disclose double stranded hairpin constructs for suppressing multiple targets. See also Sijen et al., The Plant Cell, Vol. 8, 2277-2294 (1996) which discloses the use of constructs carrying inverted repeats (sense followed by anti-sense) of a cowpea mosaic virus gene in transgenic plants to mediate virus resistance. See also International Publication No. 98/53083 (Grierson et al.) and related U.S. Patent Application Publication No. 2003/0175965 A1 (Lowe et al.) which disclose gene suppression, using a double stranded RNA construct comprising a gene coding sequence preceded by an inverted repeat of 5'UTR. Constructs for posttranscriptional gene suppression in plants by double-stranded RNA of the target gene are also disclosed in International Publication No. WO 99/53050 (Waterhouse et al.) and International Publication No. WO 99/49029 (Graham et al.). See also U.S. Patent Application Publication No. 2002/0048814 A1 (Oeller) where DNA constructs are transcribed to sense or anti-sense RNA with a hairpin-forming poly(T)-poly(A) tail. See also U.S. Patent Application Publication No. 2003/0018993 A1 (Gutterson et al.) where sense or anti-sense DNA is followed by an inverted repeat of the 3' untranslated region of the NOS gene. See also U.S. Patent Application Publication No. 2003/0036197 A1 (Glassman et al.) where RNA for reducing the expression of target mRNA comprises a part with homology to target mRNA and a part with complementary RNA regions that are unrelated to endogenous RNA.

The production of dsRNA in plants to inhibit gene expression, e.g. in a nematode feeding on the plant, is disclosed U.S. Patent 6,506,559 (Fire et al.). Multi-gene suppression vectors for use in plants are disclosed in U.S. Patent Application No.10/465,800 (Fillatti).

Transcriptional suppression such as promoter *trans* suppression can be affected by a expressing a DNA construct comprising a promoter operably linked to inverted repeats of promoter DNA from a target gene. Constructs useful for such gene suppression mediated by promoter *trans* suppression are disclosed by Mette et al., The EMBO Journal, Vol. 18, pp. 241-148, (1999) and by Mette et al., The EMBO Journal, Vol. 19, pp. 5194-5201-148, (2000).

### Summary

This invention provides methods and recombinant DNA constructs for suppression of at least one target gene in plant cells which comprises in 5' to 3' order a promoter element active in plant cells, operably linked to an anti-sense-oriented DNA element from at least one target gene and a sense-oriented DNA element comprising 50 to 5000 nucleotides, wherein the sense-oriented DNA element is shorter than the anti-sense-oriented DNA element, and sense-oriented RNA transcribed by the sense-oriented DNA is complementary to the 5'-most end of anti-sense-oriented RNA transcribed by the anti-sense-oriented DNA element, wherein said transcribed RNA forms a loop of anti-sense-oriented RNA for suppressing said at least one target gene, and wherein said anti-sense-oriented DNA element comprises, in series, segments from two or more genes targeted for suppression.

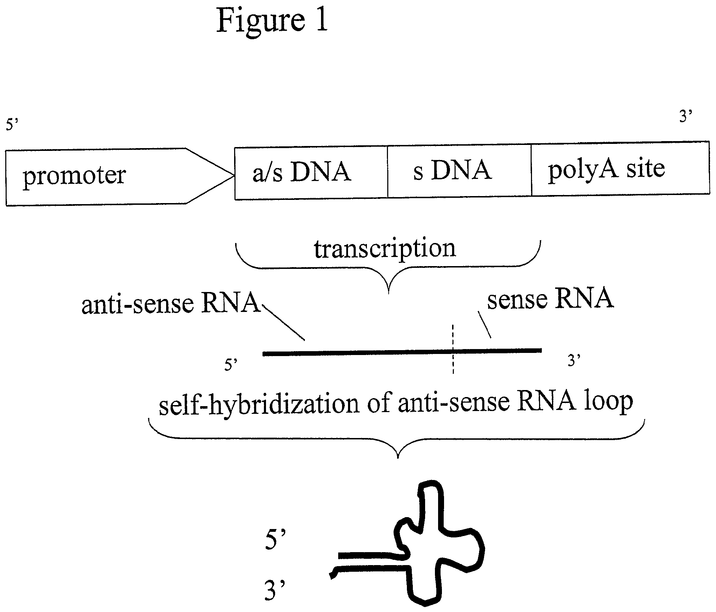

The sense-oriented DNA can be cloned as an inverted repeat of 5'-most segment of the anti-sense-oriented DNA element. Constructs with such sense-oriented DNA are transcribed to RNA that forms a loop of anti-sense-oriented RNA closed at its ends with a double-stranded RNA (dsRNA) segment, e.g. as illustrated in Figure 1. To form an anti-sense-oriented RNA loop the complementary DNA element is conveniently not more than about one-half the length of the anti-sense-oriented DNA element, often not more than one-third the length of said anti-sense-oriented DNA element, e.g. not more than one-quarter the length of said anti-sense-oriented DNA element. The overall lengths of the combined DNA elements can vary. For instance, the anti-sense-oriented DNA element can consist of from 500 to 5000 nucleotides and the complementary DNA element can consist of from 50 to 500 nucleotides. In many cases it is useful for the anti-sense-oriented DNA segment to be more than twice the length of the sense-oriented DNA segment to allow for formation of an anti-sense-oriented RNA loop.

The anti-sense transcription unit can be designed to suppress multiple genes where the DNA is arranged with two or more anti-sense-oriented elements from different genes targeted for suppression followed by a complementary sense-oriented element, e.g. complementary to at least a part of the 5'most anti-sense element.

This invention further provides a method for generating anti-sense oriented RNA in a plant for suppression of a target gene, said method comprising providing in cells of said plant a recombinant DNA construct of this invention.
In constructs and methods of this invention the gene targeted for silencing can be a native gene or an exogenous gene.

### Brief Description of the Drawing

Figure 1 is a schematic illustration of a recombinant DNA construct useful in this invention to produce an anti-sense-oriented loop of RNA.
Figure 2 is a Western analysis indicating gene suppression using a construct of this invention.
Figure 3 shows mass spectroscopy spectra indicating zein content in seeds.

### Detailed Description

SEQ ID NO:1 and SEQ ID NO:2 are nucleotide sequences of recombinant DNA constructs useful for transcribing RNA that can form an anti-sense-oriented RNA loop for suppressing one or multiple genes in transgenic plants. See Tables 1 and 2 for a description of elements of those constructs. As used herein, "complementary" refers to polynucleotides that are capable of hybridizing, e.g. sense and anti-sense strands of DNA or self-complementary strands of RNA, due to complementarity of aligned nucleotides permitting C-G and A-T or A-U bonding.

As used herein "vector" means a DNA molecule capable of replication in a host cell and/or to which another DNA segment can be operatively linked so as to bring about replication of the attached segment. A plasmid is an exemplary vector.

As used herein a "transgenic" plant or seed, is one whose genome has been altered by the incorporation of recombinant DNA comprising exogenous genetic material or additional copies of native genetic material, e.g. by transformation or recombination of the plant. Transgenic plants include progeny plants of an original plant derived from a transformation process including progeny of breeding transgenic plants with wild type plants or other transgenic plants.
Crop plants of particular interest in the present invention include maize, soybean, cotton, canola (rape), wheat, rice, sunflower, safflower and flax. Other crops of interest include plants producing vegetables, fruit, grass and wood.

### Recombinant DNA Constructs For Plant Transformation

Recombinant DNA constructs for producing looped, anti-sense RNA, gene suppression agents in transgenic plants can be readily prepared by those skilled in the art. Typically, such a DNA construct comprises as a minimum a promoter active in the tissue targeted for suppression, a transcribable DNA element having a sequence that is complementary to nucleotide sequence of a gene targeted for suppression and a transcription terminator element. The targeted gene element copied for use in transcribable DNA in the gene suppression construct can be a promoter element, an intron element, an exon element, a 5' UTR element, or a 3'UTR element. Although the minimum size of DNA copied from sequence of a gene targeted for suppression is believed to be about 21 or 23 nucleotides; larger nucleotide segments are preferred, e.g. up the full length of a targeted gene. Useful lengths of either DNA segment are in the range of 50 to 5000 nucleotides, say anti-sense-oriented DNA of 500 to 5000 nucleotides in length and complementary DNA elements can be 50 to 500 or more nucleotides in length. The DNA element can comprise multiple parts of a gene, e.g. nucleotides that are complementary to contiguous or separated gene elements of UTR, exon and intron. Such constructs may also comprise other regulatory elements, DNA encoding transit peptides, signal peptides, selective markers and screenable markers as desired.

With reference to Figure 1 there is schematically shown a recombinant DNA construct comprising a promoter element, an anti-sense-oriented DNA element (denoted "a/s DNA"), a complementary sense-oriented DNA element (denoted "s DNA") and DNA providing polyadenylation signals and site (denoted "polyA site"). The DNA construct is transcribed to RNA comprising an anti-sense-oriented RNA segment and a complementary RNA segment which is complementary to the 5'-most end of the anti-sense-oriented RNA segment. The 5' and 3' ends of the anti-sense RNA can self hybridize to form a double-stranded RNA segment that closes a loop of anti-sense-oriented RNA. For example, if the nucleotide sequence of the 5'-most end of the strand of transcribed anti-sense-oriented DNA is 5'-CGGCATA---, the sequence of the 3'-most end of the transcribed strand of the inverted repeat DNA will be ---TATGCCG-3' which is readily cloned from the source DNA providing the anti-sense element. With such sequences the loop of anti-sense-oriented RNA will extend from one side of a dsRNA segment, e.g.
5'-GCCGUAU--------
3'-CGGCAUA--------

The anti-sense-oriented DNA and its self-complementary DNA can be contiguous or separated by vector DNA, e.g. up to about 100 nucleotides or so of vector DNA separating restriction sites used for vector assembly.

Recombinant DNA constructs can be assembled using commercially available materials and methods known to those of ordinary skill in the art. A useful technology for building DNA constructs and vectors for transformation is the GATEWAY™ cloning technology (available from Invitrogen Life Technologies, Carlsbad, California) uses the site specific recombinase LR cloning reaction of the Integrase *att* system from bacterophage lambda vector construction, instead of restriction endonucleases and ligases. The LR cloning reaction is disclosed in U.S. Patents 5,888,732 and 6,277,608, U.S. Patent Application Publications 2001283529, 2001282319 and 20020007051.

The GATEWAY™ Cloning Technology Instruction Manual which is also supplied by Invitrogen also provides concise directions for routine cloning of any desired DNA into a vector comprising operable plant expression elements.

An alternative vector fabrication method employs ligation-independent cloning as disclosed by Aslanidis, C. et al., Nucleic Acids Res., 18, 6069-6074, 1990 and Rashtchian, A. et al., Biochem., 206, 91-97,1992 where a DNA fragment with single-stranded 5' and 3' ends are ligated into a desired vector which can then be amplified *in vivo.*

Numerous promoters that are active in plant cells have been described in the literature. These include promoters present in plant genomes as well as promoters from other sources, including nopaline synthase (nos) promoter and octopine synthase (ocs) promoters carried on tumor-inducing plasmids of *Agrobacterium tumefaciens,* caulimovirus promoters such as the cauliflower mosaic virus or figwort mosaic virus promoters. For instance, see U.S. Patents 5,322,938 and 5,858,742 which disclose versions of the constitutive promoter derived from cauliflower mosaic virus (CaMV35S), US Patent 5,378,619 which discloses a Figwort Mosaic Virus (FMV) 35S promoter, U.S. Patent 5,420,034 which discloses a napin promoter, U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. Patent 6,635,806 which discloses a coixin promoter, U.S. 2002/0192813A1 which discloses 5', 3' and intron elements useful in the design of effective plant expression vectors, U.S.2004/0216189 A1 which discloses a maize chloroplast aldolase promoter, and U.S. 2004/0123347A1 which discloses water-deficit inducible promoters.

These and numerous other promoters that function in plant cells are known to those skilled in the art and available for use in recombinant polynucleotides of the present invention to provide for expression of desired genes in transgenic plant cells.

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted upstream (5') or downstream (3') to the coding sequence. In some instances, these 5' enhancing elements are introns. Particularly useful as enhancers are the 5' introns of the rice actin 1 (see US Patent 5,641,876) and rice actin 2 genes, the maize alcohol dehydrogenase gene intron, the maize heat shock protein 70 gene intron (U.S. Patent 5,593,874) and the maize shrunken 1 gene.

Sufficient expression in plant seed tissues is also desired to effect improvements in seed composition. Exemplary promoters for use for seed composition modification include promoters from seed genes such as napin (U.S. 5,420,034), maize L3 oleosin (U.S. 6,433,252), zein Z27 (Russell et al. (1997) Transgenic Res. 6(2):157-166), globulin 1 (Belanger et al (1991) Genetics 129:863-872), glutelin 1 (Russell (1997) *supra),* and peroxiredoxin antioxidant (Perl) (Stacy et al. (1996) Plant Mol Biol. 31(6):1205-1216).

Recombinant DNA constructs in accordance with the invention may often include a 3' element that typically contains a polyadenylation signal and site, especially if the recombinant DNA is intended for protein expression as well as gene suppression. Well-known 3' elements include those from *Agrobacterium tumefaciens* genes such as *nos 3', tml 3', trnr 3', tms 3', ocs 3', tr7 3',* e.g. disclosed in U.S. 6,090,627 ; 3' elements from plant genes such as wheat *(Triticum aesevitum)* heat shock protein 17 *(Hspl 73'),* a wheat ubiquitin gene, a wheat fructose-1,6-biphosphatase gene, a rice glutelin gene a rice lactate dehydrogenase gene and a rice beta-tubulin gene, all of which are disclosed in U.S. published patent application 2002/0192813 A1 ; and the pea *(Pisum sativum)* ribulose biphosphate carboxylase gene *(rbs 3),* and 3' elements from the genes within the host plant.

The gene-suppressing recombinant DNA constructs can also be stacked with DNA imparting other traits of agronomic interest including DNA providing herbicide resistance or insect resistance such as using a gene from *Bacillus thuringiensis* to provide resistance against lepidopteran, coliopteran, homopteran, hemiopteran, and other insects. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and glufosinate herbicides. Persons of ordinary skill in the art are enabled in providing stacked traits by reference to U.S. patent application publications 2003/0106096A1 and 2002/0112260A1 and U.S. Patents 5,034,322; 5,776,760; 6,107,549 and 6,376,754 and to insect/nematode/virus resistance by reference to U.S. Patents 5,250,515; 5,880,275; 6,506,599; 5,986,175 and U.S. Patent Application Publication 2003/0150017 A1.

Transformation Methods - Numerous methods for transforming plant cells with recombinant DNA are known in the art and may be used with the recombinant DNA construct and method of the present invention. Two commonly used methods for plant transformation are *Agrobacterium-mediated* transformation and microprojectile bombardment. Microprojectile bombardment methods are illustrated in U.S. Patents 5,015,580 (soybean); 5,550,318 (corn); 5,538,880 (com); 5,914,451 (soybean); 6,160,208 (corn); 6,399,861 (com) and 6,153,812 (wheat) and Agrobacterium-mediated transformation is described in U.S. Patents 5,159,135 (cotton); 5,824,877 (soybean); 5,591,616 (corn); and 6,384,301 (soybean).

For *Agrobacterium tumefaciens* based plant transformation system, additional elements present on transformation constructs will include T-DNA left and right border sequences to facilitate incorporation of the recombinant polynucleotide into the plant genome.

In general it is useful to introduce recombinant DNA randomly, i.e. at a nonspecific location, in the genome of a target plant line. In special cases it may be useful to target recombinant DNA insertion in order to achieve site-specific integration, e.g. to replace an existing gene in the genome, to use an existing promoter in the plant genome, or to insert a recombinant polynucleotide at a predetermined site known to be active for gene expression. Several site specific recombination systems exist which are known to function in plants include cre-lox as disclosed in U.S. Patent 4,959,317 and FLP-FRT as disclosed in U.S. Patent 5,527,695.
Transformation methods which are of interest in the context of this invention are preferably practiced in tissue culture on media and in a controlled environment. "Media" refers to the numerous nutrient mixtures that are used to grow cells *in vitro,* that is, outside of the intact living organism. Recipient cell targets include meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, immature embryos, seedling apical meristems and microspores. Cells capable of
proliferating as callus are also recipient cells for genetic transformation. Practical transformation methods and materials for making transgenic plantsdisclosed herein, e.g. various media and recipient target cells, transformation of immature embryos and subsequent regeneration of fertile transgenic plants are disclosed in U.S. Patents 6,194,636 and 6,232,526.

The seeds of transgenic plants can be harvested from fertile transgenic plants and be used to grow progeny generations of transformed plants disclosed herein including hybrid plant lines for screening of plants having an enhanced agronomic trait. In addition to direct transformation of a plant with a recombinant DNA, transgenic plants can be prepared by crossing a first plant having a recombinant DNA with a second plant lacking the DNA. For example, recombinant DNA can be introduced into a first plant line that is amenable to transformation to produce a transgenic plant which can be crossed with a second plant line to introgress the recombinant DNA into the second plant line. A transgenic plant with recombinant DNA providing an enhanced agronomic trait, e.g. enhanced yield, can be crossed with a transgenic plant line having other recombinant DNA that confers another trait, e.g. herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA Progeny plants carrying DNA for both parental traits can be crossed back into the female parent line multiple times, e.g. usually 6 to 8 generations, to produce a progeny plant with substantially the same genotype as one original transgenic parental line but for the recombinant DNA of the other transgenic parental line.

In the practice of transformation DNA is typically introduced into only a small percentage of target cells in any one transformation experiment. Marker genes are used to provide an efficient system for identification of chose cells that are stably transformed by receiving and integrating a transgenic DNA construct into their genomes. Preferred marker genes provide selective markers which confer resistance to a selective agent, such as an antibiotic or herbicide. Any of the herbicides to which plants disclosed herein may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin and paromomycin *(nptll),* hygromycin B *(aph IV)* and gentamycin *(aac3* and *aacC4)* or resistance to herbicides such as glufosinate *(bar or pat)* and glyphosate *(aroA* or EPSPS). Examples of such selectable markers are illustrated in U.S. Patents 5,550,318; 5,633,435; 5,780,708 and 6,118,047.

Screenable markers which provide an ability to visually identify transformants can also be employed, *e.g.,* a gene expressing a colored or fluorescent protein such as a luciferase or green fluorescent protein (GFP) or a gene expressing a beta-glucuronidase or *uidA* gene (GUS) for which various chromogenic substrates are known.

Cells that survive exposure to the selective agent, or cells that have been scored positive in a screening assay, may be cultured in regeneration media and allowed to mature into plants. Developing plantlets can be transferred to plant growth mix, and hardened off, *e.g.,* in an environmentally controlled chamber at about 85% relative humidity, 600 ppm CO₂, and 25-250 microeinsteins m⁻²s⁻¹ of light, prior to transfer to a greenhouse or growth chamber for maturation. Plants are regenerated from about 6 weeks to 10 months alter a transformant is identified, depending an the initial tissue. Plants may be pollinated using conventional plant breeding methods known to those of skill in the art and seed produced, e.g. self-pollination is commonly used with transgenic corn. The regenerated transformed plant or its progeny seed or plants can be tested for expression of the recombinant DNA and screened for the presence of enhanced agronomic trait.

### Transgenic Plants and Seeds

Transgenic plant seed disclosed herein are grown to generate transgenic plants having an enhanced trait as compared to a control plant. Such seed for plants with enhanced agronomic trait is identified by screening transformed plants or progeny seed for enhanced trait. For efficiency a screening program is designed to evaluate multiple transgenic plants (events) comprising the recombinant DNA, e.g. multiple plants from 2 to 20 or more transgenic events.
Transgenic plants grown from transgenic seed provided herein demonstrate improved agronomic traits that contribute to increased yield or other trait that provides increased plant value, including, for example, improved seed quality. Of particular interest are plants having enhanced yield resulting from improved plant growth and development, stress tolerance, improved seed development, higher light response, improved flower development, or improved carbon and/or nitrogen metabolism

Many transgenic events which survive to fertile transgenic plants that produce seeds and progeny plants will not exhibit an enhanced agronomic trait. Screening is necessary to identify the transgenic plant having enhanced agronomic traits from populations of plants transformed as described herein by evaluating the trait in a variety of assays to detect an enhanced agronomic trait. These assays also may take many forms, including analyses to detect changes in the chemical composition, biomass, physiological properties, morphology of the plant.

The following examples illustrate aspects of the specification.

### Example 1

This example illustrates preparation of a transformation vector useful for inserting a recombinant DNA construct of this invention into a transgenic plant to practice a method of this invention.
The *LKR*/*SDH* gene encodes a pre-protein for lysine ketoglutarate reductase (LKR) and saccharopine dehydrogenase (SDH) which are enzymes in a lysine catabolic pathway. Suppression of *LKR* is manifest in modification, e.g. increase, of lysine content. Suppression of *LKR* is effected by expressing in a plant a recombinant DNA construct that produces a stabilized anti-sense RNA transcribed from anti-sense-oriented *LKR* DNA and sense-oriented *LKR* DNA which forms a loop of anti-sense-oriented RNA.

A transformation vector is prepared comprising two transcription units between right and left borders from *Agrobacterium tumefaciens.* One transcription unit for a marker comprised:
(a) DNA of a rice actin promoter and rice actin intron,
(b) DNA of a chloroplast transit peptide from *Arabidopsis* EPSPS
(c) DNA of *A. tumefaciens aroA* (a glyphosate-resistant marker), and
(d) DNA of *A. tumefaciens NOS* terminator,

The other transcription unit for *LKR* gene suppression comprised:
(a) DNA of *Zea mays GLB1* promoter,
(b) DNA of a *Zea mays ADH1* intron,
(c) Anti-sense-oriented DNA fragment of *Zea mays LKR,*
(d) Sense-oriented DNA fragment of *Zea mays LKR,* and
(e) DNA of *Zea mays GLB1* terminator.

SEQ ID NO: 1 is DNA sequence of a transformation vector comprising the above-described marker and gene suppression elements. See Table 1 below for a description of the elements of the transformation vector contained within SEQ ID NO:1 .

**Table 1.**

| **Bases of SEQ ID NO:1** | **Description of DNA segment** |
|---|---|
| 1-357 | *A. tumefaciens* right border |
| 376-1774 | DNA of a rice actin promoter and rice actin intron |
| 1784-2011 | DNA of *A. tumefaciens* EPSPS chloroplast transit peptide |
| 2012-3379 | DNA of *A*. *tumefaciens aroA* (glyphosate-resistant marker) |
| 3395-3647 | DNA of *A. tumefaciens NOS* terminator |
| 3691-4686 | DNA of *Zea mays Glb1* terminator |
| 4692-5145 | Sense-oriented DNA element from *Zea mays LKR* |
| 5152-6118 | Anti-sense-oriented DNA element from *Zea mays LKR* |
| 6123-6680 | DNA of a *Zea mays ADH1* intron |
| 6687-8082 | DNA of *Zea mays GLB1* promoter |
| 8149-8590 | *A. tumefaciens* left border |

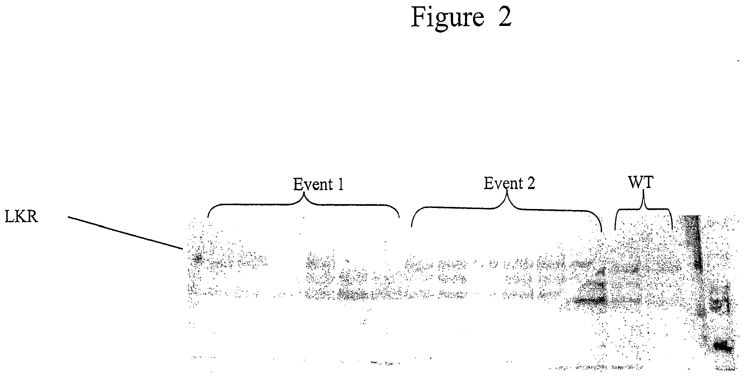

A vector prepared with the elements listed in Table 1 was used to transform corn plant tissue. Transgenic corn plants were obtained by *Agrobacterium*-mediated transformation. Transgenic plants from two separate transgenic insertion events were grown to produce F1 seed. Six mature seeds from each event were analyzed to determine success of transformation and suppression of *LKR*. The mature transgenic seeds were dissected to extract protein which was analyzed by Western analysis. With reference to Figure 2, seed from one of the events showed no reduction in *LKR* as compared to wild type; and seed from the other event was shown to be segregating (1:1 hemizygous:wild type) as three of the six seeds showed substantial reduction in *LKR* as compared to wild type.

### Example 2

This example illustrates a wide scope of transformation vectors useful for inserting a recombinant DNA construct of this invention into a transgenic plant to practice a method of this invention. Transformation vectors were prepared using the following DNA elements where:
(a) "pGcx" refers to DNA for a promoter derived from a gamma coixin gene from *Coix lacryma-jobi;*
(b) "pZ27" refers to DNA for a promoter derived from a gamma zein gene from *Zea mays;*
(c) "pZ27t" refers to DNA for a truncated promoter having 59 nucleotides leader sequence deleted from the 3' region of pZ27;
(d) "Z19as" refers to DNA for an antisense-oriented segment of 351 nucleotides from the coding sequence of a 19 kilo dalton alpha zein gene from *Zea mays;*
(e) "Z19s" refers to DNA for a sense-oriented segment of 351 nucleotides from the coding sequence of a 19 kilo dalton alpha zein gene from *Zea mays,* which is an inverted repeat of Z19as;
(f) "Z22as" refers to DNA for an antisense-oriented segment of 789 nucleotides from the coding sequence of a 22 kilo dalton alpha zein gene from *Zea mays;*
(g) "Z22asL" refers to DNA for an antisense-oriented segment of 785 nucleotides from the coding sequence of a 22 kilo dalton alpha zein gene from *Zea mays;*
(h) "Z22asSI" refers to DNA for an antisense-oriented segment of 789 nucleotides from the coding sequence of a 22 kilo dalton alpha zein gene from *Zea mays* having a 520 nucleotide long spliceable intron from a GB1 gene intron 3 from *Zea mays* inserted in the unpaired region;
(i) "Z22s" refers to DNA for a sense-oriented segment of 289 nucleotides from the coding sequence of a 22 kilo dalton alpha zein gene from *Zea mays,* which is an inverted repeat of the 5' end of Z22as; and
(j) "TE9" refers to DNA for a sense oriented polyadenylation signal and site element from an RbcS2 gene from *Pisum sativum.*

With reference to Table 2 and SEQ ID NO:2 a transformation vector comprising "construct 2a" was made in the manner of Example 1 except that the transcription unit for LKR gene suppression was replaced by a transcription unit comprising the elements illustrated in the following schematic:
"Construct 2a" pZ27 - Z19as - Z22asL - Z22s - Z19s - TE9

**Table 2**

| **Bases of SEQ ID NO:2** | **description of DNA segment** |
|---|---|
| 1-357 | *A. tumefaciens* right border |
| 376-1774 | DNA of a rice actin promoter and rice actin intron |
| 1784-2011 | DNA of *A*. *tumefaciens* EPSPS chloroplast transit peptide |
| 2012-3379 | DNA of *A*. *tumefaciens aroA* (glyphosate-resistant marker) |
| 3395-3647 | DNA of *A*. *tumefaciens NOS* terminator |
| 3479-4391 | DNA of *Pisum sativum RbcS2* terminator |
| 4398-4748 | DNA for Z19s |
| 4755-5043 | DNA for Z22s |
| 5050-5835 | DNA of Z22asL |
| 5842-6192 | DNA of Z19as |
| 6204-7305 | DNA of *Zea mays Z27* promoter |
| 7353-7794 | *A. tumefaciens* left border |

Corn callus was transformed and events with a single copy of the transformation vector were selected for growth into plants. Seed from plants grown from 26 of 29 single copy events showed substantial reduction of the 19 kilo dalton alpha zeins and the 22 kilo Dalton alpha zeins.

Other transformation vectors were made in a similar manner using the elements illustrated in the following Table 3.

**Table 3**

| | |
|---|---|
| Construct 2b1 | pGcx - Z19as - Z22asSI - Z22s - Z19s - TE9 |
| Construct 2b2* | pGcx - Z19as - Z22asSI - Z22s - Z19s - TE9 |
| Construct 2c | pZ27 - Z19as - Z22asSI - Z22s - Z19s - TE9 |
| Construct 2d | PZ27t - Z19as - Z22asSI - Z22s - Z19s - TE9 |
| Construct 2e | PZ27 - Z19as - Z22asL - Z 19s - TE9 |

| | |
|---|---|
| * construct 2b2 was inserted into a transformation vector that also included a transcription unit for expressing another gene having a promoter contiguous to pGcx. | |

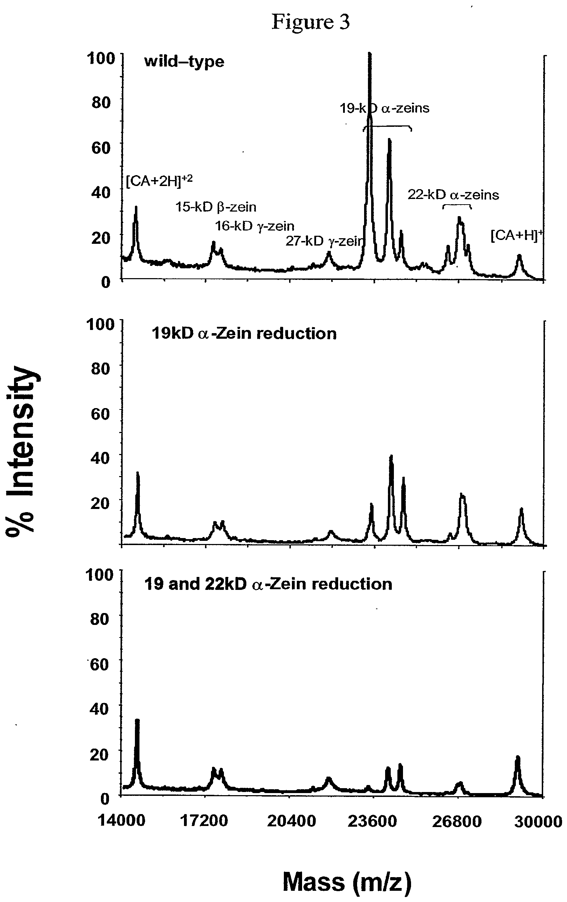

The efficiency of suppressing the alpha zeins in seeds produced by plants grown from single copy events is reported in Table 4 which reports the number of transgenic events with reduction of zeins as compared to the total number of transgenic events generated in each construct tested. The zein reduction phenotype is observed by MALDI-TOF MS (Matrix-Assisted-Laser-Desorption Ionization Time-Of-Flight Mass Spectrometry) analysis. Figure 3 is illustrates typical spectra evidencing zein reduction.

**Table 4**

| Construct | 19 kD zein | 19 and 22 kD zein |
|---|---|---|
| 2a | 26/29 | 26/29 |
| 2b1 | 0/21 | 0/21 |
| 2b2 | 5/7 | 0/7 |
| 2c | 20/21 | 18/21 |
| 2d | 7/8 | 1/8 |
| 2e | 12/14 | 2/14 |

## Claims

1. A recombinant DNA construct for suppression of at least one target gene in plant cells which comprises in 5' to 3' order a promoter element active in plant cells, operably linked to an anti-sense-oriented DNA element from at least one target gene and a sense-oriented DNA element comprising 50 to 5000 nucleotides, wherein the sense-oriented DNA element is shorter than the anti-sense-oriented DNA element, and sense-oriented RNA transcribed by the sense-oriented DNA is complementary to the 5'-most end of anti-sense-oriented RNA transcribed by the anti-sense-oriented DNA element, wherein said transcribed RNA forms a loop of anti-sense-oriented RNA for suppressing said at least one target gene, and wherein said anti-sense-oriented DNA element comprises, in series, segments from two or more genes targeted for suppression.

2. The recombinant DNA construct of claim 1, wherein said loop of anti-sense-oriented RNA is closed with a segment of double-stranded RNA.

3. The recombinant DNA construct of claim 2, wherein one strand of said segment of double-stranded RNA is identical to mRNA from a gene targeted for suppression.

4. The recombinant DNA construct of claim 1, wherein said sense-oriented DNA element comprises from 50 to 500 nucleotides.

5. The recombinant DNA construct of claim 1, wherein said sense-oriented DNA element is not more than about one-third the length of the anti-sense-oriented DNA element.

6. The recombinant DNA construct of claim 1, wherein said sense-oriented DNA element is not more than about one-quarter the length of the anti-sense-oriented DNA element.

7. A method for generating anti-sense-oriented RNA in a plant for suppression of a target gene, said method comprising providing in cells of said plant a recombinant DNA construct according to any one of claims 1 to 6.

## Patentansprüche

1. Rekombinantes DNA-Konstrukt zur Unterdrückung wenigstens eines Zielgens in Pflanzenzellen, das in der Reihenfolge von 5' nach 3' ein in Pflanzenzellen aktives Promotorelement, das funktionell mit einem Antisense-orientierten DNA-Element von wenigstens einem Zielgen und einem Sense-orientierten DNA-Element, das 50 bis 5000 Nucleotide umfasst, verknüpft ist, wobei das Sense-orientierte DNA-Element kürzer ist als das Antisense-orientierte DNA-Element und die von der Sense-orientierten DNA transkribierte Sense-orientierte RNA zu dem 5'-Ende der von dem Antisense-orientierten DNA-Element transkribierten Antisense-orientierten RNA komplementär ist, wobei die transkribierte RNA eine Schleife aus Antisense-orientierter RNA bildet, um das wenigstens eine Zielgen zu unterdrücken, und wobei das Antisense-orientierte DNA-Element in Reihe Segmente aus zwei oder mehr Genen, die Zielgene für die Unterdrückung sind, umfasst.

2. Rekombinantes DNA-Konstrukt gemäß Anspruch 1, wobei die Schleife aus Antisense-orientierter RNA mit einem Segment von doppelsträngiger RNA geschlossen ist.

3. Rekombinantes DNA-Konstrukt gemäß Anspruch 2, wobei ein Strang des Segments der doppelsträngigen RNA mit mRNA aus einem Zielgen für die Unterdrückung identisch ist.

4. Rekombinantes DNA-Konstrukt gemäß Anspruch 1, wobei das Sense-orientierte DNA-Element 50 bis 500 Nucleotide umfasst.

5. Rekombinantes DNA-Konstrukt gemäß Anspruch 1, wobei das Sense-orientierte DNA-Element nicht mehr als etwa ein Drittel der Länge des Antisense-orientierten DNA-Elements aufweist.

6. Rekombinantes DNA-Konstrukt gemäß Anspruch 1, wobei das Sense-orientierte DNA-Element nicht mehr als etwa ein Viertel der Länge des Antisense-orientierten DNA-Elements aufweist.

7. Verfahren zur Erzeugung von Antisense-orientierter RNA in einer Pflanze zur Unterdrückung eines Zielgens, wobei das Verfahren das Bereitstellen eines rekombinanten DNA-Konstrukts gemäß einem der Ansprüche 1 bis 6 in Zellen der Pflanze umfasst.

## Revendications

1. Construction d'ADN recombiné pour supprimer au moins un gène cible dans une cellule végétale qui comprend dans le sens 5' à 3' un élément de promoteur actif dans les cellules végétales, fonctionnellement lié à un élément d'ADN orienté antisens provenant au moins d'un gène cible et à un élément d'ADN orienté sens comprenant de 50 à 5 000 nucléotides, dans laquelle l'élément d'ADN orienté sens est plus court que l'élément d'ADN orienté antisens, et l'ARN orienté sens transcrit par l'ADN orienté sens est complémentaire de la majorité de l'extrémité 5' de l'ARN orienté antisens transcrit par l'élément d'ADN orienté antisens, où ledit ARN transcrit forme une boucle d'ARN orienté antisens pour supprimer ledit au moins gène cible, et où ledit élément d'ADN orienté antisens comprend, en série, des segments provenant de deux gènes ou plus ciblés à des fins de suppression.

2. Construction d'ADN recombiné selon la revendication 1, dans laquelle ladite boucle d'ARN orienté antisens est fermée avec un segment d'ARN double brin.

3. Construction d'ADN recombiné selon la revendication 2, dans laquelle un brin dudit segment d'ARN double brin est identique à l'ARNm provenant d'un gène ciblé à des fins de suppression.

4. Construction d'ADN recombiné selon la revendication 1, dans laquelle ledit élément d'ADN orienté sens comprend de 50 à 500 nucléotides.

5. Construction d'ADN recombiné selon la revendication 1, dans laquelle ledit élément d'ADN orienté sens a une longueur n'excédant pas environ un tiers celle de l'élément d'ADN orienté antisens.

6. Construction d'ADN recombiné selon la revendication 1, dans laquelle ledit élément d'ADN orienté sens a une longueur n'excédant pas environ un quart celle de l'élément d'ADN orienté antisens.

7. Procédé de génération d'un ARN orienté antisens chez une plante pour supprimer un gène cible, ledit procédé comprenant l'introduction dans les cellules de ladite plante d'une construction d'ADN recombiné selon l'une quelconque des revendications 1 à 6.
